# EUROPÄISCHE PATENTANMELDUNG

(11) **EP 3 058 886 A1**
(43) Veröffentlichungstag der Anmeldung: **24.08.2016**
(21) Anmeldenummer: 16152190.1
(22) Anmeldetag: 21.01.2016
(51) Int. Cl.: A61B 17/88, B05C 17/01, A61B 17/00

(54) **AUSTRAGSVORRICHTUNG FÜR ZEMENTKARTUSCHEN MIT FEDERZUNGEN**

(30) Priorität: 20.02.2015 DE 102015102463
(71) Anmelder: Heraeus Medical GmbH, 61273 Wehrheim (DE)
(72) Erfinder: Dr. Vogt, Sebastian, 99092 Erfurt (DE)
(74) Vertreter: Heraeus IP

(57) **Zusammenfassung**

Austragsvorrichtung (1) aufweisend mindestens ein manuell zu betätigendes Bedienelement (4), einen Adapter (44) für eine Zementkartusche und eine in Richtung des Adapters vortreibbare Klemmstange (2), wobei an der Klemmstange anliegend zumindest ein Vortriebskörper (24) angeordnet ist, der mehrere elastische Federzungen (50) aufweist, die jeweils eine Schneidkante (54) aufweisen. Die Federzungen liegen über die Schneidkante an der Klemmstange an und sind gegen die Klemmstange derart geneigt, dass bei einer Bewegung des Vortriebskörpers in Vortriebsrichtung der Klemmstange die Schneidkanten des Vortriebskörpers in die Klemmstange greifen. Ein Federelement (40) wirkt zumindest zeitweise mit einer elastischen Kraft entgegengesetzt zur Vortriebsrichtung der Klemmstange auf den zumindest einen Vortriebskörper, wobei über das Bedienelement eine Kraft auf den zumindest einen Vortriebskörper in Vortriebsrichtung der Klemmstange auszuüben ist. Ein Sicherungselement (34) liegt an der Klemmstange an, gegen das der Vortriebskörper beweglich gelagert ist. Das Sicherungselement weist mehrere elastische Federzungen (52) auf, die jeweils eine Schneidkante (56) aufweisen.

## Beschreibung

Die Erfindung betrifft eine Austragsvorrichtung für Zementkartuschen für Polymethylmethacrylat-Knochenzemente aufweisend mindestens ein manuell zu betätigendes Bedienelement, einen Adapter für eine Zementkartusche und eine in Richtung des Adapters vortreibbare Klemmstange.

Gegenstand der Erfindung ist somit eine manuell angetriebene Vorrichtung zum Austrag von Polymethylmethacrylat-Knochenzementteig aus Kartuschen von Vakuumzementiersystemen. Die Vorrichtung ist zum nur einmaligen Gebrauch bestimmt und soll nach der Verwendung thermisch wiederverwertet werden können (verbrannt werden können).

Gelenkendoprothesen werden in der Orthopädie und Unfallchirurgie im breiten Umfang zum Ersatz von menschlichen Gelenken verwendet, wenn diese durch Krankheiten, Unfall oder durch Verschleiß geschädigt sind. Die dauerhafte mechanische Fixierung von Gelenkendoprothesen erfolgt dabei durch mechanische Klemmung (press-fit) oder durch Zementierung mit Polymethylmethacrylat-Knochenzementen (PMMA-Knochenzementen).

PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, häufig einen Röntgenopaker und den Initiator Dibenzoylperoxid auf. Vor der Zementapplikation wird die Pulverkomponente mit der flüssigen Monomerkomponente vermischt. Beim Vermischen entsteht durch Quellung der Polymere der Pulverkomponente im Methylmethacrylat ein plastisch verformbarer Teig, der eigentliche Knochenzement. Dabei reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt. Der erstarrte Polymethylmethacrylat-Knochenzement ist mechanisch stabil und kann Gelenkendoprothesen dauerhaft im Knochengewebe der Patienten befestigen.

Die Komponenten von Polymethylmethacrylat-Knochenzementen (PMMA-Knochenzementen) können in geeigneten Mischbechern mit Hilfe von Spateln vermischt werden. Nachteilig ist an dieser Vorgehensweise, dass Lufteinschlüsse im gebildeten Zementteig vorhanden sein können, die später eine Destabilisierung des ausgehärteten Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offengelegt, von denen exemplarisch folgende genannt sind: US 6 033 105 A, US 5 624 184 A, US 4 671 263 A, US 4 973 168 A, US 5 100 241 A, WO 99/67015 A1, EP 1 020 167 B1, US 5 586 821 A, EP 1 016 452 A2, DE 36 40 279 C2, WO 94/26403 A1, EP 1 005 901 A2, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden in den Patenten EP 0 692 229 B1, DE 10 2009 031 178 B3, EP 0 875 456 B3, US 6 709 149 B1 und EP 1 140 234 B1 sowie der US 5 588 745 A vorgeschlagen.

Bei der Verwendung von Vakuum-Zementiersystemen ist es zur Applikation notwendig, den Zementteig durch Bewegung eines Kolbens aus den Zementkartuschen auszupressen. Dazu wurden manuelle Austragsvorrichtungen entwickelt.

In der EP 0 326 551 A1 wurde ein interessantes Hebelsystem für manuell angetriebene Vorrichtungen beschrieben. Die Idee dieses Hebelsystems basiert darauf, ein Hebelparallelogramm zu nutzen, um im Gegensatz zu einem einfachen Kipphebel die Kraft der stärksten Finger der menschlichen Hand, dem Zeigefinger und dem Mittelfinger, optimal zu nutzen.

Die einfachsten Austragsvorrichtungen basieren auf Klemmstangen mit einem darauf angeordneten kippbaren Metallplättchen, das durch eine asymmetrisch angreifende Feder verkantet und dadurch die Klemmstange einklemmt. Das kippbare Metallplättchen wird durch einen Hebel bei manueller Betätigung in Vortriebsrichtung gepresst, wobei das klemmende Metallblättchen die Klemmstange mitnimmt. Danach schiebt eine Feder das Metallplättchen zurück in seine Ausgangslage. Dieser Prozess wird wiederholt bis die Klemmstange den Förderkolben des Vakuum-Zementiersystems so weit in Richtung Kartuschenkopf gepresst hat, bis die gewünschte Knochenzementmenge aus der Kartusche gepresst ist.

Nachteilig an diesen Vorrichtungen ist, dass bei der Klemmung immer eine Rückwärtsbewegung durch Rutschen des Metallplättchens auf der Klemmstange möglich ist. Durch ein Zurückrutschen des Metallplättchens benötigt der Anwender wesentlich mehr Handbewegungen zum Auspressen des Knochenzementteigs, als eigentlich notwendig wären.

Eine Weiterentwicklung stellen Austragsvorrichtungen dar, die Zahnstangen anstelle einfacher Klemmstangen verwenden. Exemplarisch dafür ist die Austragsvorrichtung gemäß Figur 28der Patentanmeldung US 2013 090 661 A1. Vorteilhaft ist bei solchen Vorrichtungen, dass eine Rückwärtsbewegung praktisch ausgeschlossen ist. Nachteilig ist jedoch die komplexe, aufwendige Mechanik, die eine Verwendung dieser Vorrichtung zum nur einmaligen Gebrauch hinsichtlich der relativ hohen Herstellungskosten in Frage stellt.

Die Aufgabe der Erfindung besteht darin, die Nachteile des Stands der Technik zu überwinden. Insbesondere soll eine einfach zu fertigende Austragsvorrichtung bereitgestellt werden, mit der ein Polymethylmethacrylat-Knochenzementteig aus Zementkartuschen von Vakuumzementiersystemen manuell ausgepresst werden kann. Die manuell angetriebene Vorrichtung soll nur zum einmaligen Gebrauch geeignet und bestimmt sein. Die Austragsvorrichtung soll weitgehend aus kostengünstigen Kunststoffteilen, die durch Kunststoffspritzgießen hergestellt werden können, und möglichst wenigen Metallelementen bestehen. Insbesondere soll die Austragsvorrichtung mit einer aus Kunststoff gefertigten Klemmstange aufgebaut werden. Eine Klemmstange aus Metall, insbesondere Stahl, soll möglichst vermieden werden. Durch annähernd vollständigen Aufbau der Vorrichtung aus Kunststoffen, kann diese nach der einmaligen Verwendung thermisch wieder verwertet werden (in einer Müllverbrennungsanlage verbrannt werden), ohne dass größer Mengen an Metall anfallen beziehungsweise verloren gehen.

Bei der manuellen Betätigung der Austragsvorrichtung soll eine Rückwärtsbewegung des Förderkolbens der Kartusche während des Auspressvorgangs infolge von elastischen Rückstellkräften der Kunststoffkartusche und des Zementteigs so weit wie möglich vermieden werden. Die zu entwickelnde Austragsvorrichtung soll nur ein einmaliges Austragen von Polymethylmethacrylat-Knochenzementteig ermöglichen. Eine Wiederverwendung und dadurch notwendige Resterilisation der Austragsvorrichtung soll konstruktiv ausgeschlossen sein.

Die Aufgaben der Erfindung werden gelöst durch eine Austragsvorrichtung für Zementkartuschen für Polymethylmethacrylat-Knochenzemente aufweisend mindestens ein manuell zu betätigendes Bedienelement, einen Adapter für eine Zementkartusche und eine in Richtung des Adapters vortreibbare Klemmstange, wobei an der Klemmstange anliegend zumindest ein Vortriebskörper angeordnet ist, der in axialer Richtung, bezüglich der Achse der Klemmstange, in der Austragsvorrichtung verschiebbar ist,
wobei der zumindest eine Vortriebskörper mehrere elastische Federzungen aufweist, wobei die Federzungen jeweils eine Schneidkante aufweisen, wobei die Federzungen über die Schneidkante an der Klemmstange anliegen, wobei die Schneidkanten der Federzungen oder die gesamten Federzungen eine größere Härte als die Klemmstange aufweisen und wobei die Federzungen des Vortriebskörpers derart gegen die Klemmstange geneigt sind, dass bei einer Bewegung des Vortriebskörpers in Vortriebsrichtung der Klemmstange die Schneidkanten des Vortriebskörpers in die Klemmstange greifen und dass bei einer Bewegung des Vortriebskörpers entgegen der Vortriebsrichtung der Klemmstange die Federzungen des Vortriebskörpers derart elastisch verformbar sind, dass die Schneidkanten über die Klemmstange gleiten,
wobei ein Federelement zumindest zeitweise mit einer elastischen Kraft entgegengesetzt zur Vortriebsrichtung der Klemmstange auf den zumindest einen Vortriebskörper wirkt,
wobei über das Bedienelement eine Kraft auf den zumindest einen Vortriebskörper in Vortriebsrichtung der Klemmstange auszuüben ist, wobei mit dieser Kraft der Vortriebskörper und die Klemmstange gegen die elastische Kraft des Federelements relativ zum Adapter vorzutreiben ist,
wobei ein Sicherungselement an der Klemmstange anliegt, gegen das der Vortriebskörper beweglich gelagert ist,
wobei das Sicherungselement mehrere elastische Federzungen aufweist, wobei die Federzungen jeweils eine Schneidkante aufweisen, wobei die Federzungen über die Schneidkante an der Klemmstange anliegen, wobei zumindest die Schneidkanten der Federzungen oder die gesamten Federzungen eine größere Härte als die Klemmstange aufweisen und wobei die Federzungen derart gegen die Klemmstange geneigt sind, dass bei einer Bewegung der Klemmstange entgegen der Vortriebsrichtung die Schneidkanten des Sicherungselements in die Klemmstange greifen und dass bei einer Bewegung der Klemmstange in Vortriebsrichtung die Federzungen des Sicherungselements derart elastisch verformbar sind, dass die Schneidkanten über die Klemmstange gleiten.

Wenn die Klemmstange in Richtung des Adapters vortreibbar ist, bedeutet dies im Rahmen der vorliegenden Erfindung, dass die Klemmstange auch durch den Adapter hindurch vorzutreiben ist, auch wenn der Schwerpunkt der Klemmstange sich dann gegen Ende des Austragsvorgangs tatsächlich von dem Adapter weg bewegt. Mit der Achse der Klemmstange ist die geometrische Achse der Klemmstange gemeint und nicht etwa eine Drehachse.

Erfindungsgemäß ist bevorzugt vorgesehen, dass die Austragsvorrichtung zum manuellen Auspressen von Zementkartuschen geeignet ist, die mit Polymethylmethacrylat-Knochenzement oder mit Ausgangskomponenten für PMMA-Knochenzement gefüllt sind. Besonders bevorzugt ist die Austragsvorrichtung mit einer Hand zu halten und mit der gleichen Hand zu bedienen. Zum Auspressen muss die Zementkartusche an den Adapter der Austragsvorrichtung angeschlossen sein.

Die Schneidkanten können erfindungsgemäß durch eine ebene Kante, durch eine gewellte Kante oder durch eine Sägezahnartige Kante oder auch durch Spitzen gebildet sein. Die Schneidkante muss dabei nicht im eigentlichen Sinne in die Klemmstange einschneiden, sondern kann sich auch eindrücken oder in einer anderen Art in die Klemmstange eingreifen, indem die Klemmstange von der Schneidkante plastisch verformt wird.

Wenn die Schneidkanten in die Klemmstange greifen, bedeutet dies, dass die Schneidkanten in die Klemmstange einschneiden, eindrücken oder die Schneidkanten anderweitig in die Oberfläche der Klemmstange eindringen. Dabei kommt es zu einer plastischen Verformung der Klemmstange, insbesondere an deren Oberfläche.

Bevorzugt bewirkt das Federelement, insbesondere drückt das Federelement, in jeder Stellung des zumindest einen Vortriebskörpers mit der elastischen Kraft entgegengesetzt zur Vortriebsrichtung der Klemmstange auf den zumindest einen Vortriebskörper. Es kann bei mehreren Vortriebskörpern auch vorgesehen sein, dass das Federelement mehrere einzelne Federn aufweist, die jeweils einzeln oder in Gruppen auf die verschiedenen Vortriebskörper wirken.

Mit der axialen Richtung ist vorliegend immer die Richtung bezeichnet, entlang derer sich die Achse der Klemmstange erstreckt und bewegen lässt.

Anstatt einer in Richtung des Adapters vortreibbaren Klemmstange kann theoretisch auch eine in Wirkrichtung vortreibbare Klemmstange verwendet werden, wenn die Kraft, die durch den Vortrieb der Klemmstange entsteht, über Gelenke, Zahnräder oder ähnliches umgelenkt wird, dabei die Wirkrichtung geändert wird und sich dort der Adapter zum Anschließen der Zementkartusche befindet, und sich daher die Klemmstange nicht mehr geometrisch in Richtung des Adapters vortreiben lässt. Dies ist als äquivalenter Aufbau mit gleichem Wirkprinzip zu verstehen.

Mit der Erfindung wird auch vorgeschlagen, dass der zumindest eine Vortriebskörper in axialer Richtung gegen die Klemmstange und gegen den Adapter linear beweglich ist und/oder das Sicherungselement unbeweglich gegen den Adapter mit der Austragsvorrichtung verbunden ist.

Hierdurch ergibt sich ein besonders einfacher und kostengünstig zu realisierender Aufbau der Austragsvorrichtung.

Des Weiteren kann vorgesehen sein, dass bei einer Bewegung der Klemmstange entgegen der Vortriebsrichtung die Schneidkanten der Federzungen des Sicherungselements derart in die Klemmstange greifen, dass eine weitere Bewegung der Klemmstange entgegen der Vortriebsrichtung verhindert ist.

Hierdurch wird sichergestellt, dass ein Zurückdrücken der Klemmstange verhindert wird. Dies kann dadurch entstehen, dass beim Auspressen der Zementkartusche sich diese elastisch verformt oder Gas in der Zementkartusche komprimiert wird und dadurch eine Kraft von der Zementkartusche oder deren Inhalt auf die Klemmstange ausgeübt wird, die gegen die Vortriebsrichtung der Klemmstange wirkt, auch wenn die Klemmstange nicht weiter zum oder durch den Adapter vorangetrieben wird. Zudem wird so verhindert, dass sich der angetriebene Förderkolben in der Zementkartusche zurück bewegt.

Gemäß einer bevorzugten Weiterbildung der Erfindung kann vorgesehen sein, dass die Schneidkanten der Federzungen des zumindest einen Vortriebskörpers derart in die Klemmstange greifen, dass eine Bewegung der Klemmstange mit dem zumindest einen Vortriebskörper in Vortriebsrichtung erfolgt.

Damit wird erreicht, dass die Klemmstange effektiv mit dem Bedienelement über den Vortriebskörper vorgetrieben werden kann.

Ferner kann erfindungsgemäße auch vorgesehen sein, dass die elastischen Federzungen des zumindest einen Vortriebskörpers und des Sicherungselements derart geneigt sind, dass die Schneidkanten in Richtung der Vortriebsrichtung ausgerichtet sind und sich die weiterführenden Teile der Federzungen ausgehend von den Schneidkanten entgegen der Vortriebsrichtung erstrecken, wobei bevorzugt die Federzungen mit einem Winkel zwischen 88° und 20° gegen die Achse der Klemmstange geneigt sind, besonders bevorzugt mit einem Winkel zwischen 80° und 70° gegen die Achse der Klemmstange geneigt sind.

Hierdurch wird erreicht, dass die Federzungen bei einer geeigneten Bewegung der Klemmstange gegen das Sicherungselement oder bei einer geeigneten Bewegung des Vortriebskörpers in die Klemmstange greifen, das heißt, die Klemmstange einklemmen, da die Federzungen in dieser Richtung nicht oder nur schwer elastisch verformbar sind, und bei einer entgegengesetzten Bewegungsrichtung die Schneidkanten aufgrund einer elastischen Deformation der Federzungen über die Klemmstange gleiten, das heißt, die Bewegung der Klemmstange nicht behindern.

Bevorzugte Austragsvorrichtungen können sich auch dadurch auszeichnen, dass die elastischen Federzungen des zumindest einen Vortriebskörpers und des Sicherungselements derart an der Klemmstange anliegen, dass die Schneidkanten mit einer elastischen Kraft der elastisch verformten Federzungen auf die Klemmstange gedrückt sind.

Damit wird sichergestellt, dass die Schneidkanten der Federzungen immer in Kontakt mit der Klemmstange sind und bei geeigneter Bewegung in die Oberfläche der Klemmstange direkt eingreifen können.

Des Weiteren wird mit der Erfindung vorgeschlagen, dass das manuell zu betätigende Bedienelement ein manuell zu betätigender Kipphebel ist, wobei der Kipphebel drehbar gegen die Klemmstange gelagert ist, so dass bei Betätigung des Kipphebels ein Ende des Kipphebels auf den zumindest einen axial verschiebbaren Vortriebskörper in Vortriebsrichtung der Klemmstange drückt.

Hierdurch kann eine Hebelkraft eingesetzt werden, um den Vortriebskörper und damit die Klemmstange mit großer Kraft vorzudrücken. Bevorzugt ist dabei der manuell zu bedienende Teil des Kipphebels weiter vom Drehpunkt entfernt, als der Teil des Kipphebels, der auf den Vortriebskörper wirkt, bevorzugt zumindest doppelt so weit. Hierdurch wird eine Vergrößerung der Kraft ermöglicht. Wenn kein Kipphebel als Bedienelement verwendet wird, muss eine andere Kraftübersetzung für das Bedienelement gefunden werden, um zähe PMMA-Zemente aus der Zementkartusche auspressen zu können. Erfindungsgemäß ist eine Kraftübersetzung von mindestens 2:1 bevorzugt, besonders bevorzugt von mindestens 4:1. Das bedeutet, dass bei einem vollständigen Hub des Bedienelements die Klemmstange nur um die Hälfte des Hubs beziehungsweise ein Viertel des Hubs vorangetrieben wird.

Unter einem drehbar gelagerten Kipphebel wird vorliegend ein Kipphebel verstanden, der um einige Grad gegen die Klemmstange zu drehen ist. Bevorzugt ist der Kipphebel um 20° bis 70° gegen die Klemmstange zu drehen.

Der Kipphebel ist bevorzugt als Hebelsystem gemäß der EP 0 326 551 A1 ausgebildet. Bei diesem Hebelsystem wird ein manuell zu betätigendes Hebelparallelogramm verwendet, das eine kraftvolle Betätigung des Hebels durch den Zeigefinger und Mittelfinger ermöglicht.

Bei bevorzugten Austragsvorrichtungen kann vorgesehen sein, dass die elastischen Federzungen an der Innenseite von zumindest einem ringförmigen Federplättchen angeordnet sind, wobei das zumindest eine Federplättchen die Klemmstange vollständig oder zu mehr als 50% umschließt.

Hierdurch kann der Aufbau weiter vereinfacht werden, da das ringförmige Federplättchen als einheitliches Bauteil in dem Vortriebskörper und/oder dem Sicherungselement eingebaut werden können. Zudem sind die Federplättchen kostengünstig zu fertigen beziehungsweise kostengünstig käuflich zu erwerben.

Es wird mit der Erfindung auch vorgeschlagen, dass der zumindest eine an der Klemmstange anliegende Vortriebskörper mit der Klemmstange zumindest eine Kavität bildet und/oder das an der Klemmstange anliegende Sicherungselement mit der Klemmstange zumindest eine Kavität bildet, wobei in der Kavität oder in den Kavitäten die Federzungen angeordnet sind, insbesondere das zumindest eine ringförmige Federplättchen angeordnet ist.

Hierdurch kann der Aufwand bei dem Zusammenbau der Austragsvorrichtung vereinfacht werden, ohne dass die Funktionalität der Austragsvorrichtung darunter leiden würde.

Dabei kann vorgesehen sein, dass die durch den Vortriebskörper und/oder die durch das Sicherungselement gebildete Kavität auf der zum Adapter ausgerichteten Seite durch ein Verschlusselement, insbesondere einen Ring, verschlossen ist.

Dies ist insbesondere dann vorteilhaft, wenn die Federzungen oder Federplättchen in die Kavitäten eingesetzt werden, da mit den Verschlusselementen beziehungsweise den Ringen die Kavitäten vorne (aus Richtung des Adapters) verschlossen werden und dadurch ein herausfallen der Federzungen oder der Federplättchen verhindert werden kann. Bevorzugt ist das zumindest eine Verschlusselement elastisch, insbesondere besteht das zumindest eine Verschlusselement aus einem elastischen Kunststoff. Dadurch können die Federzungen elastisch in die Kavitäten gedrückt und positioniert werden.

Gemäß einer Weiterbildung der erfindungsgemäßen Austragsvorrichtung kann auch vorgesehen sein, dass die Austragsvorrichtung ein Gehäuse mit einem Handgriff aufweist, wobei vorzugsweise das Sicherungselement fest mit dem Gehäuse verbunden ist und der zumindest eine Vortriebskörper linear beweglich gegen das Gehäuse gelagert ist.

Hierdurch wird die Position des zumindest einen Sicherungselements mit dem Gehäuse festgelegt, so dass es keiner separaten Lagerung des Sicherungselements bedarf, was den Aufbau der Austragsvorrichtung vereinfacht.

Ferner kann vorgesehen sein, dass der zumindest eine Vortriebskörper die Klemmstange zumindest bereichsweise umschließt, bevorzugt zu mindestens 75% umschließt, besonders bevorzugt vollständig umschließt.

Dadurch kann die Kraft besonders einfach allseitig auf die Klemmstange wirken. Zudem wird so effektiv verhindert, dass die Austragsvorrichtung demontiert werden kann, so dass dieser Aufbau bevorzugt wird.

Dabei kann wiederum vorgesehen sein, dass der zumindest eine Vortriebskörper eine Durchführung aufweist, die bevorzugt als Röhre ausgebildet ist.

Hierdurch kann ein Verkippen und Verkanten des Vortriebskörpers auf der Klemmstange vermieden werden.

Alternativ dazu kann auch vorgesehen sein, dass mehrere axial verschiebbare Vortriebskörper aus unterschiedlichen Richtungen an der Klemmstange anliegen.

Der einzelne Vortriebskörper kann so einfacher aufgebaut werden. Allerdings wird dadurch das Zusammensetzen der Austragsvorrichtung erschwert und gleichzeitig eine unerwünschte Demontage der Austragsvorrichtung erleichtert.

Bevorzugte Ausführungsformen können sich dadurch auszeichnen, dass die Schneidkanten, insbesondere die Federzungen oder das zumindest eine Federplättchen, eine Härte von mindestens 45 HRC haben, vorzugsweise von mindestens 50 HRC haben.

Hierdurch kann ein mehrfaches Festziehen und wieder lösen der Federzungen von der Klemmstange sichergestellt werden. Unter dem Begriff "HRC" wird die Rockwell-Härte nach der Skala C gemäß der DIN EN ISO 6508-1 verstanden.

Ferner wird vorgeschlagen, dass die Klemmstange aus einem Kunststoff besteht, bevorzugt aus einem Duroplast.

Hierdurch kann die Austragsvorrichtung im Wesentlichen kostengünstig aus Kunststoff gefertigt werden und ist so als hygienischer Einmalartikel verwendbar. Besonders bevorzugt besteht die Klemmstange aus einem Kunststoff der Gruppe der Phenolharze, der Harnstoffharze, der Polyurethane und der Epoxide.

Es kann vorgesehen sein, dass das zumindest eine Sicherungselement auf der Klemmstange vor oder auch hinter dem zumindest einen Vortriebskörper in Bezug zur Vortriebsrichtung der Klemmstange angeordnet ist.

Bevorzugt ist das Sicherungselement auf der Klemmstange zwischen dem zumindest einen Vortriebskörper und dem Adapter angeordnet.

Ferner wird vorgeschlagen, dass die Federzungen, insbesondere die Federplättchen, aus Federstahl bestehen.

Federstahl ist einerseits ausreichend hart und stabil, um in eine Klemmstange aus Kunststoff eingreifen zu können und dabei nicht zu brechen, und andererseits sehr elastisch, um die notwendige elastische Verformbarkeit bereitzustellen, wenn die Schneidkanten über die Klemmstange gleiten sollen. Zudem sind die genannten Bauteile aus Federstahl günstig zu fertigen oder käuflich zu erwerben.

Die der vorliegenden Erfindung zugrundeliegenden Aufgaben werden auch gelöst, durch ein Verfahren zum Vortreiben einer Klemmstange mit einem manuell zu bedienenden Bedienelement bei dem durch manuelle Krafteinwirkung auf das Bedienelement zumindest ein an der Klemmstange anliegender Vortriebskörper in einer ersten axialen Richtung der Klemmstange bewegt wird,
wobei Schneidkanten mehrerer Federzungen des zumindest einen Vortriebskörpers in die Klemmstange eingreifen und dadurch gegen die Klemmstange klemmen, so dass die Klemmstange mit dem Vortriebskörper in die erste axialen Richtung bewegt wird,
wobei, sobald die manuelle Krafteinwirkung auf das Bedienelement reduziert wird oder beendet wird, der zumindest eine Vortriebskörper durch ein Federelement in eine der ersten axialen Richtung entgegengesetzte zweite axiale Richtung gedrückt wird, wobei das Bedienelement wieder in die Ausgangsstellung überführt wird und wobei sich durch die umgekehrte Krafteinwirkung die Schneidkanten der Federzungen des zumindest einen Vortriebskörpers über die Klemmstange gleiten, und
wobei eine umgekehrte Bewegung der Klemmstange in die zweite axiale Richtung durch zumindest ein an der Klemmstange anliegendes Sicherungselement blockiert wird, wobei hierzu Schneidkanten von Federzungen des Sicherungselements durch die umgekehrte Bewegung der Klemmstange in die Klemmstange eingreifen und dadurch gegen die Klemmstange klemmen, so dass die Klemmstange sich nicht mehr gegen das zumindest eine Sicherungselement bewegt.

Die Schritte erfolgen dabei erfindungsgemäß bevorzugt chronologisch in der angegebenen Reihenfolge.

Wenn von einer Blockade der Bewegung der Klemmstange durch das Sicherungselement die Rede ist, muss dies dahingehend eingeschränkt werden, dass es bei Eingreifen der Schneidkanten der Federzungen des Sicherungselements selbstverständlich zu einer geringen Rückwärtsbewegung der Klemmstange kommt, bis die Bewegung endgültig von dem Sicherungselement blockiert wird. Wie weit sich die Klemmstange zurückbewegen kann hängt von der ausgeübten rücktreibenden Kraft, der Anzahl und der Länge der Schneidkanten, der Lagerung der Klemmstange, der elastischen Verformbarkeit der Federzungen entgegen der Vortriebsrichtung und von der Härte der Klemmstange ab.

Für erfindungsgemäße Verfahren wird vorgeschlagen, dass die Oberfläche der Klemmstange durch die Schneidkanten der Federzungen des Vortriebskörpers und des Sicherungselements plastisch verformt wird, wenn die Schneidkanten der Federzungen des Vortriebskörpers und des Sicherungselements in die Klemmstange greifen.

Hierdurch kann eine stabile Verbindung der Federzungen mit der Klemmstange erreicht werden.

Erfindungsgemäß kann auch vorgesehen sein, dass sich bei einer Bewegung der Klemmstange in die erste axiale Richtung die Schneidkanten der Federzungen des zumindest einen Sicherungselements von der Klemmstange aus den durch die Schneidkanten erzeugten Vertiefungen in der Klemmstange lösen und die Klemmstange dadurch gegen das zumindest eine Sicherungselement bewegt wird.

Damit wird sichergestellt, dass sich die Klemmstange einfach vortreiben lässt. Die Schneidkanten liegen dann aufgrund der Federkraft der Federzungen noch immer an der Klemmstange an, greifen aber nicht mehr in die Klemmstange ein.

Es wird des Weiteren vorgeschlagen, dass beim manuellen Bedienen des Bedienelements, insbesondere beim Kippen eines Kipphebels als Bedienelement, durch die manuelle Krafteinwirkung das Federelement komprimiert wird.

Hierdurch wird das Federelement für die anschließende Rückstellung des zumindest einen Vortriebskörpers gespannt.

Bevorzugte Ausführungsformen können vorsehen, dass die Bedienung des Bedienelements mehrfach wiederholt wird und dabei die Klemmstange schubweise vorgetrieben wird und dabei schubweise ein Knochenzement aus einer Zementkartusche ausgetrieben wird, die zuvor über einen Adapter mit einer Austragsvorrichtung verbunden wurde, wobei die Austragsvorrichtung den Adapter, das Bedienelement, die Klemmstange, den zumindest einen Vortriebskörper und das Sicherungselement umfasst.

Hierdurch wird erreicht, dass durch wiederholtes Bedienen des Bedienelements die Klemmstange schrittweise vorangetrieben wird, so dass bei einer einzigen Bedienung ausreichend Kraft zum Austreiben des oft zähen Knochenzements vorhanden ist. Die Zementkartusche kann als einzelne Kartusche oder als Kartuschensystem vorliegen. An einer Austragsöffnung der Zementkartusche kann ein statischer Mischer vorgesehen sein, der den Austrag des Knochenzements weiter erschwert.

Schließlich kann vorgesehen sein, dass das Verfahren durch Verwendung oder unter Anwendung einer erfindungsgemäßen Austragsvorrichtung durchgeführt wird.

Der Erfindung liegt die überraschende Erkenntnis zugrunde, dass es durch die Verwendung eines Sicherungselements und eines manuell angetriebenen Vortriebskörpers, die relativ zueinander beweglich an einer Klemmstange angeordnet sind und die jeweils geneigte Federzungen aufweisen, wobei die Federzungen bei einer Bewegung des Vortriebskörpers oder der Klemmstange in die Klemmstange eingreifen und diese dadurch mit dem Vortriebskörper bewegt wird oder durch das Sicherungselement an einer Bewegung gehindert wird, gelingt, einen einfachen aber effektiven unidirektionalen Vortrieb der Klemmstange zu erreichen, ohne dass die Klemmstange aufgrund elastischer Kräfte der PMMA-Zementkartusche wieder zurückgetrieben werden könnte. Die Klemmstange wird dabei zudem durch die Federzungen plastisch verformt und ein Zurückschieben der Klemmstange durch den Eingriff der Federzungen des Sicherungselements in die Klemmstange verhindert. Hierdurch wird die Wiederverwendbarkeit eingeschränkt beziehungsweise unmöglich gemacht, so dass die Verwendung nicht sterilisierter Austragsvorrichtungen unterbunden werden kann. Gleichzeitig ist der gesamte Aufbau einschließlich der Klemmstange der Austragsvorrichtung kostengünstig zu realisieren, so dass das Wegwerfprodukt nicht zu teuer in der Herstellung ist.

Es wurde also überraschend gefunden, dass mit der erfindungsgemäßen Austragsvorrichtung trotz der Verwendung einfacher, kostengünstiger Klemmstangen und von kostengünstigen Kunststoffteilen ein Austrag von Polymethylmethacrylat-Knochenzementteig möglich ist, ohne dass unerwünschte Rückwärtsbewegungen der Austragskolben während des Austragens von Polymethylmethacrylat-Knochenzementteig aus Vakuum-Zementiersystemen auftreten. Weiterhin wurde gefunden, dass es ohne Zerstörung der Austragsvorrichtung nicht möglich ist, diese in den Ausgangszustand zu versetzen, so dass eine Wiederverwendung der Vorrichtung nach erfolgtem Austrag ausgeschlossen ist.

Eine erfindungsgemäße Austragsvorrichtung kann beispielsweise zusammengesetzt sein aus mindestens einem manuell zu betätigenden Kipphebel (als Bedienelement), einer Klemmstange, einem Adapter für die Zementkartusche und einem Gehäuse mit Handgriff. Die Austragsvorrichtung kann beispielsweise dadurch charakterisiert sein, dass
a) auf der Klemmstange ein axial verschiebbarer Vortriebskörper angeordnet ist, der die Klemmstange zumindest bereichsweise umschließt,
b) der Vortriebskörper mindestens eine Kavität besitzt,
c) in der Kavität eine ringförmiges Federplättchen angeordnet ist, das an seiner Innenseite Federzungen besitzt, die in Richtung der Vortriebsbewegung der Kunststoffklemmstange ausgerichtet sind und die an der Klemmstange aufliegen,
d) ein elastisches Element (als Verschluss der Kavität), das ringförmige Federplättchen in die Kavität des Vortriebskörpers drückt,
e) der Kipphebel drehbar so im Gehäuse angeordnet ist, dass bei Betätigung des Kipphebels ein Ende des Kipphebels auf den axial verschiebbaren Vortriebskörper in Vortriebsrichtung der Kunststoffklemmstange gedrückt wird,
f) ein Federelement entgegengesetzt zur Vortriebsrichtung der Kunststoffklemmstange auf den Vortriebskörper drückt,
g) mit dem Gehäuse ein Sicherungselement verbunden ist, dass die Kunststoffklemmstange umschließt, wobei das Sicherungselement mindestens eine keilförmige Kavität besitzt, und
h) in der Kavität des Sicherungselements eine ringförmiges Federplättchen angeordnet ist, das an seiner Innenseite Federzungen besitzt, die in Richtung der Vortriebsbewegung der Kunststoffklemmstange ausgerichtet sind und die an der Klemmstange aufliegen.

Das Gehäuse und der Adapter für die Zementkartusche bestehen vorzugsweise aus Kunststoff. Der Kipphebel besteht entweder aus Kunststoff, Aluminiumlegierungen oder Stahl.

Der Kipphebel ist bevorzugt als Hebelsystem gemäß der EP 0 326 551 A1 ausgebildet. Bei diesem Hebelsystem wird ein manuell zu betätigendes Hebelparallelogramm verwendet, das eine kraftvolle Betätigung des Hebels durch den Zeigefinger und Mittelfinger ermöglicht.

Die Durchführung des Vortriebskörpers ist bevorzugt als Röhre ausgebildet. Dadurch ist ein Verkippen oder Verkanten des Vortriebskörpers auf der Klemmstange ausgeschlossen.

Erfindungsgemäß kann vorgesehen sein, dass die Federplättchen aus Federstahl gebildet sind. Die Federplättchen sind bevorzugt konisch geformt, wobei die Federzungen konisch nach innen zeigend ausgebildet sind.

Ein erfindungsgemäßes Verfahren zum Austragen von Polymethylmethacrylat-Knochenzement mit einer erfindungsgemäßen Austragsvorrichtung kann beispielsweise dadurch charakterisiert werden, dass
in einem Schritt a) manuell der Kipphebel gegen den in einer Ausgangsstellung befindlichen Vortriebskörper bewegt wird, wobei der Vortriebskörper mit Federplättchen gegen die Kunststoffklemmstange klemmt und diese in Vortriebsrichtung bewegt wird, wobei gleichzeitig das Federelement komprimiert wird,
in einem Schritt b) nach Beendigung der Vortriebsbewegung des Kipphebels der Vortriebskörpers durch das komprimierte Federelement auf der Klemmstange zurück in die Ausgangsstellung gedrückt wird,
in einem Schritt c) das Sicherungselement durch Klemmung des Federplättchens oder der Federplättchen gegen die Kunststoffklemmstange gegen eine Bewegung entgegengesetzt zur Vortriebsrichtung gesichert wird und dass die Schritte a bis c wiederholt werden.

Die Schritte erfolgen dabei bevorzugt chronologisch.

Im Folgenden werden weitere Ausführungsbeispiele der Erfindung anhand von fünf schematisch dargestellten Figuren erläutert, ohne jedoch dabei die Erfindung zu beschränken. Dabei zeigt:
- Figur 1:: eine schematische Querschnittansicht einer erfindungsgemäßen Austragsvorrichtung;
- Figur 2:: eine schematische perspektivische Ansicht der Austragsvorrichtung nach Figur 1;
- Figur 3:: eine schematische perspektivische Querschnittansicht einer Austragsvorrichtung mit einer längeren Klemmstange;
- Figur 4:: eine schematische Explosionsdarstellung der Austragsvorrichtung nach Figur 3; und
- Figur 5:: eine schematische Ansicht eines Querschnitts eines Ausschnitts einer erfindungsgemäßen Austragsvorrichtung zur Verdeutlichung der Funktionsweise.

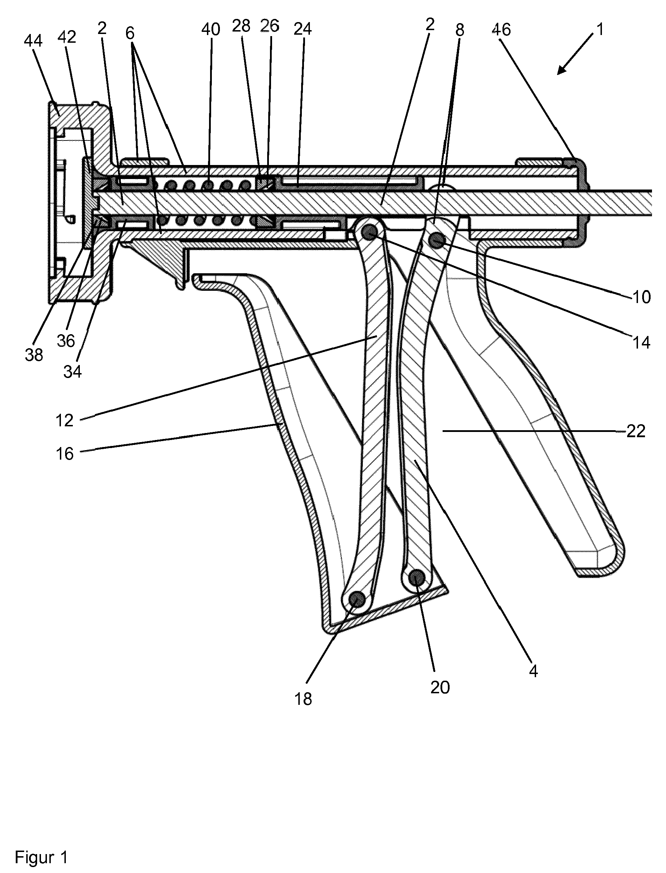

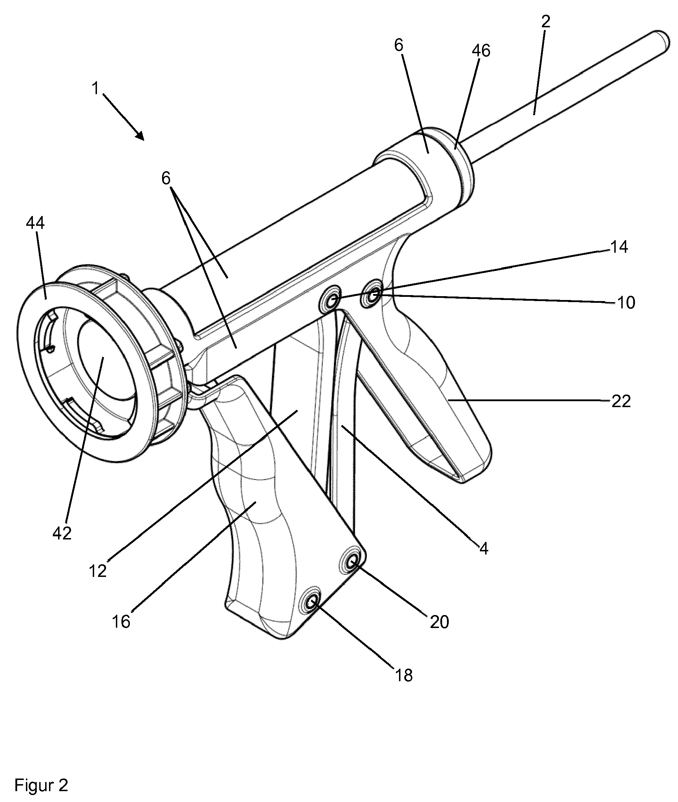

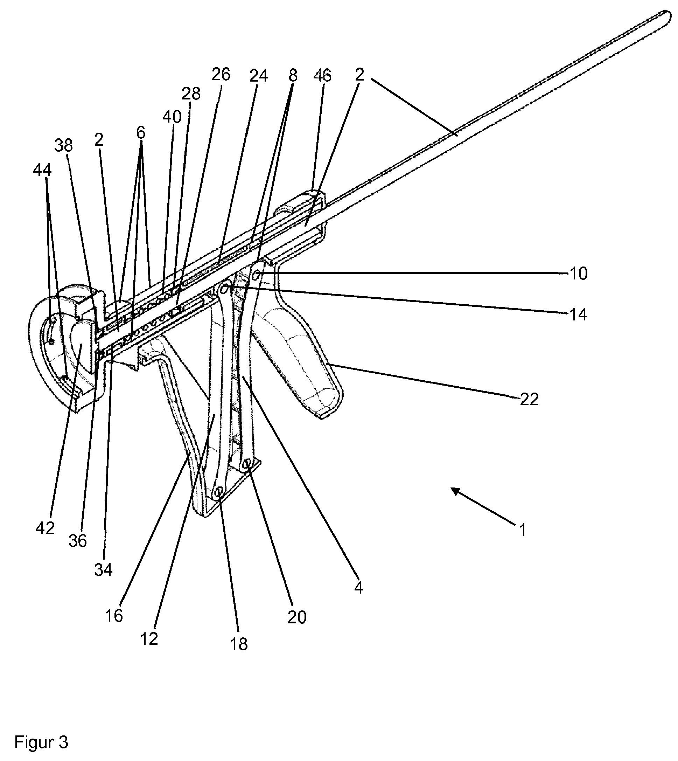

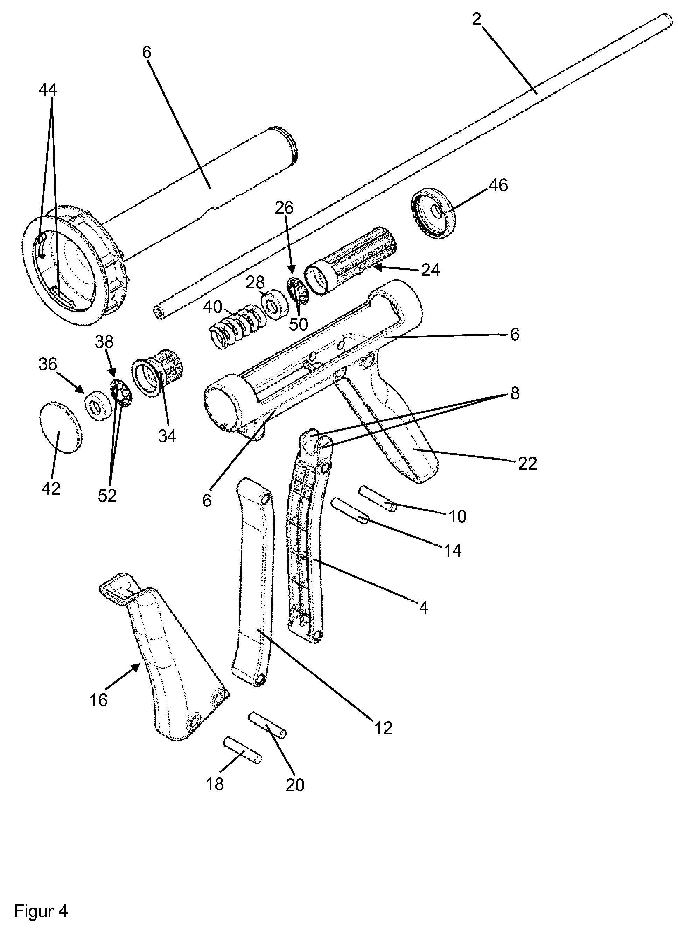

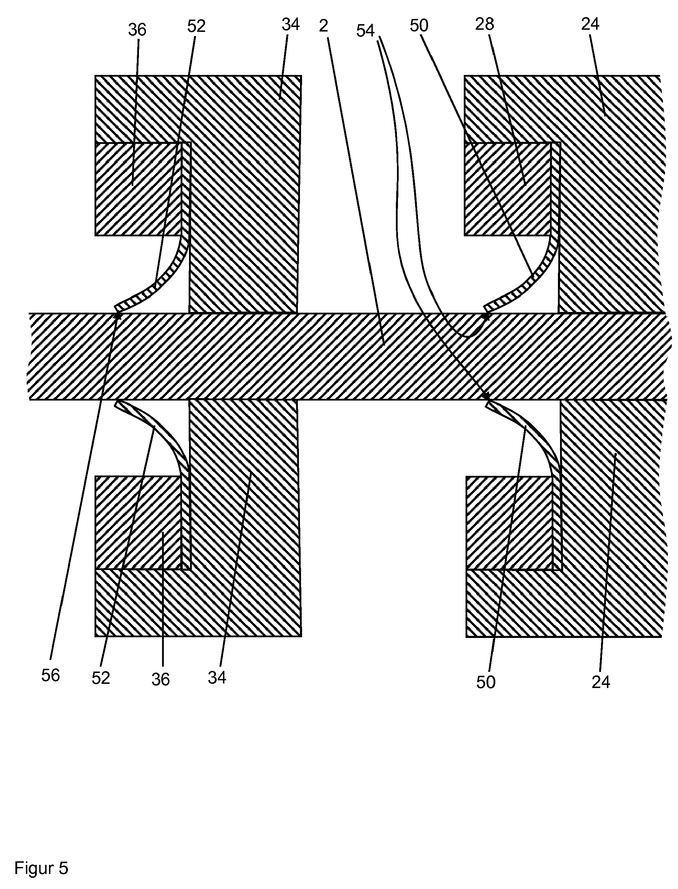

Figur 1 zeigt eine Austragsvorrichtung 1 mit einer Klemmstange 2, die nach vorne (in Figur 1 links) vortreibbar ist, in einer Querschnittansicht, wobei die geschnittenen Bereiche schraffiert dargestellt sind. Die Figur 2 zeigt eine perspektivische Außenansicht auf die Austragsvorrichtung 1 nach Figur 1. Die Figuren 3 und 4 zeigen eine perspektivische Querschnittansicht und eine Explosionsdarstellung einer weiteren Austragsvorrichtung 1, die sich gegenüber der in den Figuren 1 und 2 dargestellten Austragsvorrichtung 1 nur durch die Länge der Klemmstange 2 unterscheidet. Ein schematischer Aufbau eines Querschnitts eines Ausschnitts einer erfindungsgemäßen Austragsvorrichtung zur Verdeutlichung der Funktionsweise ist in Figur 5 dargestellt. Auch in Figur 5 sind die geschnittenen Flächen schraffiert dargestellt. In den Figuren werden die gleichen Bezugszeichen für die gleichen und gleichartigen Bauteile unterschiedlicher Austragsvorrichtungen verwendet.

Die Klemmstange 2 besteht aus einem Kunststoff und kann mit Hilfe eines Kipphebels 4 als manuell zu betätigendes Bedienelement 4 aus einem stabilen Kunststoff oder alternativ aus Stahl oder Aluminium bewegt werden. Die Austragsvorrichtung 1 wird von einem mehrteiligen Gehäuse 6 aus Kunststoff weitgehend umschlossen, damit der Innere Aufbau der Austragsvorrichtung 1 nicht offenliegt. Die Gehäuseteile 6 können als Spritzgussteile gefertigt werden. Der Kipphebel 4 endet in einem schwenkbaren Kopf 8, mit dem die Bewegung des Kipphebels 4 ins Innere des Gehäuses 6 der Austragsvorrichtung 1 übersetzt wird. Dazu ist der Kipphebel 4 über eine Achse 10 mit dem Gehäuse 6 verbunden. Bei einer Drehung des Kipphebels 4 um die Achse 10 (bei der Ansicht auf den Querschnitt nach Figur 1 und der perspektivischen Ansicht nach den Figuren 2 und 3 entgegen des Uhrzeigersinns) wird der Kopf 8 des Kipphebels 4 aufgrund der Hebelwirkung mit großer Kraft nach vorne (in den Figuren 1, 2 und 3 nach links) gedrückt.

Der Kipphebel 4 wird bedient, indem eine Strebe 12 bewegt wird, die über eine Achse 14 mit dem Gehäuse 6 und mit einem Abzug 16 über eine Achse 18 verbunden ist. Der Kipphebel 4 ist ebenfalls über eine Achse 20 mit dem Abzug 16 verbunden. Der Abzug 16 lässt sich aufgrund dieses Aufbaus parallel zu einem Griff 22 bewegen. Der Griff 22 ist als Teil des Gehäuses 6 aus Kunststoff gefertigt. Durch den Aufbau mit dem Kipphebel 4 und der Strebe 12 sowie deren Verbindungen / Achsen 10, 14, 18, 20 zum Gehäuse 6 und dem Abzug 16 kann die volle Höhe des Abzugs 16 verwendet werden, um einen Druck auf den Kipphebel 4 auszuüben. Dadurch lässt sich der Kipphebel 4 mit der Kraft der ganzen Hand und insbesondere auch des Zeigefingers und des Mittelfingers bedienen, wobei der Griff 22 mit der gleichen Hand gehalten wird. Die gesamte Austragsvorrichtung 1 kann also leicht mit einer Hand gehalten und bedient werden. Ein solcher Aufbau ist im Detail auch in der EP 0 326 551 A1 beschrieben. Die Achsen 10, 14, 18, 20 können aus Kunststoff gefertigt werden, da bei den wenigen Hüben des Kipphebels 4 kein nennenswerter Verschleiß auftreten kann.

Mit dem Kopf 8 des Kipphebels 4 wird ein Vortriebskörper 24 vorangetrieben (in den Figuren 1, 2, 3 und 5 nach links verschoben). Der Vortriebskörper 24 umschließt die Klemmstange 2 und weist an der Innenseite eine Ausnehmung auf, die mit der Klemmstange 2 eine Kavität bildet. In der Kavität ist ein Federplättchen 26 angeordnet, das mehrere Federzungen 50 aus Federstahl aufweist. Die Federzungen 50 sind als Klemmkörper vorgesehen und liegen federnd an der Klemmstange 2 mit Schneidkanten 54 an. In der Kavität, die durch den Vortriebskörper 24 und die Klammstange 2 sowie einen Verschluss 28 auf der Vorderseite (in den Figuren 1, 2, 3 und 5 links) begrenzt ist, sind mit dem Federplättchen 26 eine Vielzahl von Federzungen 50 angeordnet, wobei die Federzungen 50 die Klemmstange 2 umgeben.

Wenn der Kopf 8 des Kipphebels 4 aufgrund einer Drehung um die Achse 10 auf den Vortriebskörper 24 drückt, dann werden die Schneidkanten 54 der Federzungen 50 gegen die die Klemmstange 2 gepresst. Aufgrund der Ausrichtung der Federzungen 50 schneiden beziehungsweise greifen die Schneidkanten 54 in die Klemmstange 2 ein. Dadurch blockieren die Federzungen 50 und der Vortriebskörper 24 verklemmt sich beziehungsweise verkeilt sich mit der Klemmstange 2. Durch die geringe Auflagefläche (Auflagepunkte) der Schneidkanten 54 auf der Klemmstange 2 entsteht ein sehr großer Druck an den Auflageflächen, der ausreicht, um die Klemmstange 2 plastisch zu verformen. Hierzu ist die Härte der Schneidkanten 54 oder der Federzungen 50 oder des gesamten Federplättchens 26 aus Federstahl größer als die Härte der Klemmstange 2 aus Kunststoff. Die eingeschnittenen Schneidkanten 54 blockieren dann über die Federzungen 50 beziehungsweise über das Federplättchen 26 ein Vorschieben des Vortriebskörpers 24 auf der Klemmstange 2.

An der Vorderseite der Austragsvorrichtung 1 (in den Figuren 1, 3 und 5 links) ist ein Sicherungselement 34 vorgesehen, das in seinem Aufbau dem Vortriebskörper 24 gleicht, wobei das Sicherungselement 34 fest mit dem Gehäuse 6 verbunden ist, während der Vortriebskörper 24 im Gehäuse 6 beweglich gelagert ist.

Das Sicherungselement 34 umschließt die Klemmstange 2 und weist an der Innenseite eine Ausnehmung auf, die mit der Klemmstange 2 eine Kavität bildet. In der Kavität ist ein Federplättchen 36 aus Federstahl als Klemmkörper angeordnet. Mehrere Federzungen 52 des Federplättchens 36 liegen mit Schneidkanten 56 allseitig an der Klemmstange 2 an. In der Kavität, die durch das Sicherungselement 34 und die Klammstange 2 sowie einen Verschluss 38 auf der Vorderseite (in den Figuren 1, 2, 3 und 5 links) begrenzt ist, ist das Federplättchen 36 eingeschlossen und befestigt. Die in der Explosionsdarstellung nach Figur 4 gezeigten Federplättchen 26, 36 weisen jeweils sechs nach Innen weisende Federzungen 50, 52 auf, die jeweils paarweise einander gegenüberliegend angeordnet sind und die im eingebauten Zustand alle gleichsinnig in Bezug auf die Vortriebsrichtung der Klemmstange 2 geneigt sind.

Wenn die Klemmstange 2 mit dem Kipphebel 4 und dem Vortriebskörper 24 nach vorne getrieben wird, schiebt sich die Klemmstange 2 durch das Sicherungselement 34, da die Schneidkanten 56 bei dieser Bewegung über die Klemmstange 2 gleiten, weil diese Bewegung eine elastische Verformung der Federzungen 52 erlaubt. Gleichzeitig wird eine elastische Feder 40 gespannt beziehungsweise elastisch komprimiert, die zwischen dem Vortriebskörper 24 und dem Sicherungselement 34 in dem Gehäuse 6 angeordnet ist.

Wenn die Kraft auf den Kipphebel 4 nachlässt oder wegfällt, wird der Vortriebskörper 24 durch die gespannte Feder 40 in die entgegengesetzte Richtung nach hinten (in den Figuren 1, 2, 3 und 5 nach rechts) gedrückt. Bei dieser Bewegung können die Schneidkanten 54 an den Federzungen 50 des Federplättchens 26 des Vortriebskörpers 24 aufgrund der Neigung der Federzungen 50 gegen die Klemmstange 2 auf der Klemmstange 2 gleiten, da die Federzungen 50 bei dieser Bewegung leicht elastisch verformbar sind. Dadurch kann der Vortriebskörper 24 auf der Klemmstange 2 durch die Feder 40 verschoben werden.

Gleichzeitig ist es nicht möglich, die Klemmstange 2 wieder nach hinten (in den Figuren 1, 2, 3 und 5 nach rechts) ins Gehäuse 6 zu drücken, da diese Bewegung aufgrund der Neigung der Federzungen 52 des Sicherungselements 34 gegen die Klemmstange 2 mit den Schneidkanten 56 blockiert wird. Wenn also beim Auspressen von Knochenzement aus einer Kartusche (nicht gezeigt) elastische Kräfte auftreten, die einen Gegendruck auf die Klemmstange 2 ausüben, kann die Klemmstange 2 nicht wieder in das Gehäuse 6 zurück gedrückt werden.

An der Vorderseite ist an der Klemmstange 2 ein Stempel 42 befestigt, der zum Vortreiben eines Förderkolbens (nicht gezeigt) einer Zementkartusche (nicht gezeigt) vorgesehen ist. An der Vorderseite der Austragsvorrichtung 1 befindet sich ein Adapter 44 mit einem Bajonett-Verschluss zum Anschließen einer Zementkartusche. Die Zementkartusche (nicht gezeigt) kann an dem Adapter 44 befestigt werden, wobei der Boden der befestigten Zementkartusche den Förderkolben beinhaltet, der mit dem Stempel 42 in die Zementkartusche hineingedrückt werden kann. Durch das Vortreiben der Klemmstange 2 drückt der Stempel 42 den Förderkolben in die Zementkartusche, wobei dadurch der Inhalt der Zementkartusche (beispielsweise ein medizinischer PMMA-Knochenzement) aus der Zementkartusche durch eine dem Förderkolben gegenüberliegende Kartuschenöffnung herausgedrückt wird.

Auf der Rückseite ist die Austragsvorrichtung 1 durch eine Kappe 46 verschlossen. Die Kappe 46 weist eine Durchführung für die Klemmstange 2 auf und kann als Teil des Gehäuses 6 betrachtet werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Austragsvorrichtung
- 2: Klemmstange
- 4: Kipphebel / Bedienelement
- 6: Gehäuse
- 8: Kopf des Kipphebels
- 10: Achse
- 12: Strebe
- 14: Achse
- 16: Abzug
- 18: Achse
- 20: Achse
- 22: Griff
- 24: Vortriebskörper
- 26: Federplättchen
- 28: Verschluss
- 34: Sicherungselement
- 36: Federplättchen
- 38: Verschluss
- 40: Feder
- 42: Stempel
- 44: Adapter / Bajonettanschluss
- 46: Kappe
- 50: Federzunge
- 52: Federzunge
- 54: Schneidkante
- 56: Schneidkante

## Patentansprüche

1. Austragsvorrichtung (1) für Zementkartuschen für Polymethylmethacrylat-Knochenzemente aufweisend mindestens ein manuell zu betätigendes Bedienelement (4), einen Adapter (44) für eine Zementkartusche und eine in Richtung des Adapters (44) vortreibbare Klemmstange (2),
wobei an der Klemmstange (2) anliegend zumindest ein Vortriebskörper (24) angeordnet ist, der in axialer Richtung, bezüglich der Achse der Klemmstange (2), in der Austragsvorrichtung (1) verschiebbar ist,
wobei der zumindest eine Vortriebskörper (24) mehrere elastische Federzungen (50) aufweist, wobei die Federzungen (50) jeweils eine Schneidkante (54) aufweisen, wobei die Federzungen (50) über die Schneidkante (54) an der Klemmstange (2) anliegen, wobei die Schneidkanten (54) der Federzungen (50) oder die gesamten Federzungen (50) eine größere Härte als die Klemmstange (2) aufweisen und wobei die Federzungen (50) des Vortriebskörpers (24) derart gegen die Klemmstange (2) geneigt sind, dass bei einer Bewegung des Vortriebskörpers (24) in Vortriebsrichtung der Klemmstange (2) die Schneidkanten (54) des Vortriebskörpers (24) in die Klemmstange (2) greifen und dass bei einer Bewegung des Vortriebskörpers (24) entgegen der Vortriebsrichtung der Klemmstange (2) die Federzungen (50) des Vortriebskörpers (24) derart elastisch verformbar sind, dass die Schneidkanten (54) über die Klemmstange (2) gleiten,
wobei ein Federelement (40) zumindest zeitweise mit einer elastischen Kraft entgegengesetzt zur Vortriebsrichtung der Klemmstange (2) auf den zumindest einen Vortriebskörper (24) wirkt,
wobei über das Bedienelement (4) eine Kraft auf den zumindest einen Vortriebskörper (24) in Vortriebsrichtung der Klemmstange (2) auszuüben ist, wobei mit dieser Kraft der Vortriebskörper (24) und die Klemmstange (2) gegen die elastische Kraft des Federelements (40) relativ zum Adapter (44) vorzutreiben ist,
wobei ein Sicherungselement (34) an der Klemmstange (2) anliegt, gegen das der Vortriebskörper (24) beweglich gelagert ist,
wobei das Sicherungselement (34) mehrere elastische Federzungen (52) aufweist, wobei die Federzungen (52) jeweils eine Schneidkante (56) aufweisen, wobei die Federzungen (52) über die Schneidkante (56) an der Klemmstange (2) anliegen, wobei zumindest die Schneidkanten (56) der Federzungen (52) oder die gesamten Federzungen (52) eine größere Härte als die Klemmstange (2) aufweisen und wobei die Federzungen (52) derart gegen die Klemmstange (2) geneigt sind, dass bei einer Bewegung der Klemmstange (2) entgegen der Vortriebsrichtung die Schneidkanten (56) des Sicherungselements (34) in die Klemmstange (2) greifen und dass bei einer Bewegung der Klemmstange (2) in Vortriebsrichtung die Federzungen (52) des Sicherungselements (34) derart elastisch verformbar sind, dass die Schneidkanten (56) über die Klemmstange (2) gleiten.

2. Austragsvorrichtung (1) nach Anspruch 1, **dadurch gekennzeichnet, dass**
der zumindest eine Vortriebskörper (24) in axialer Richtung gegen die Klemmstange (2) und gegen den Adapter (44) linear beweglich ist und/oder das Sicherungselement (34) unbeweglich gegen den Adapter (44) mit der Austragsvorrichtung (1) verbunden ist.

3. Austragsvorrichtung (1) nach Anspruch 1 oder 2, **dadurch gekennzeichnet, dass**
bei einer Bewegung der Klemmstange (2) entgegen der Vortriebsrichtung die Schneidkanten (56) der Federzungen (52) des Sicherungselements (34) derart in die Klemmstange (2) greifen, dass eine weitere Bewegung der Klemmstange (2) entgegen der Vortriebsrichtung verhindert ist.

4. Austragsvorrichtung (1) nach einem der vorangehenden Ansprüche, **dadurch gekennzeichnet, dass**
die Schneidkanten (54) der Federzungen (50) des zumindest einen Vortriebskörpers (24) derart in die Klemmstange (2) greifen, dass eine Bewegung der Klemmstange (2) mit dem zumindest einen Vortriebskörper (24) in Vortriebsrichtung erfolgt.

5. Austragsvorrichtung (1) nach einem der vorangehenden Ansprüche, **dadurch gekennzeichnet, dass**
die elastischen Federzungen (50, 52) des zumindest einen Vortriebskörpers (24) und des Sicherungselements (34) derart geneigt sind, dass die Schneidkanten (54, 56) in Richtung der Vortriebsrichtung ausgerichtet sind und sich die weiterführenden Teile der Federzungen (50, 52) ausgehend von den Schneidkanten (54, 56) entgegen der Vortriebsrichtung erstrecken, wobei bevorzugt die Federzungen (50, 52) mit einem Winkel zwischen 88° und 20° gegen die Achse der Klemmstange (2) geneigt sind, besonders bevorzugt mit einem Winkel zwischen 80° und 70° gegen die Achse der Klemmstange (2) geneigt sind.

6. Austragsvorrichtung (1) nach einem der vorangehenden Ansprüche, **dadurch gekennzeichnet, dass**
die elastischen Federzungen (50, 52) des zumindest einen Vortriebskörpers (24) und des Sicherungselements (34) derart an der Klemmstange (2) anliegen, dass die Schneidkanten (54, 56) mit einer elastischen Kraft der elastisch verformten Federzungen (50, 52) auf die Klemmstange (2) gedrückt sind.

7. Austragsvorrichtung (1) nach einem der vorangehenden Ansprüche, **dadurch gekennzeichnet, dass**
das manuell zu betätigende Bedienelement (4) ein manuell zu betätigender Kipphebel (4) ist, wobei der Kipphebel (4) drehbar gegen die Klemmstange (2) gelagert ist, so dass bei Betätigung des Kipphebels (4) ein Ende (8) des Kipphebels (4) auf den zumindest einen axial verschiebbaren Vortriebskörper (24) in Vortriebsrichtung der Klemmstange (2) drückt.

8. Austragsvorrichtung (1) nach einem der vorangehenden Ansprüche, **dadurch gekennzeichnet, dass**
die elastischen Federzungen (50, 52) an der Innenseite von zumindest einem ringförmigen Federplättchen (26, 36) angeordnet sind, wobei das zumindest eine Federplättchen (26, 36) die Klemmstange (2) vollständig oder zu mehr als 50% umschließt.

9. Austragsvorrichtung (1) nach einem der vorangehenden Ansprüche, **dadurch gekennzeichnet, dass**
der zumindest eine an der Klemmstange (2) anliegende Vortriebskörper (24) mit der Klemmstange (2) zumindest eine Kavität bildet und/oder das an der Klemmstange (2) anliegende Sicherungselement (34) mit der Klemmstange (2) zumindest eine Kavität bildet, wobei in der Kavität oder in den Kavitäten die Federzungen (50, 52) angeordnet sind, insbesondere das zumindest eine ringförmige Federplättchen (26, 36) angeordnet ist.

10. Austragsvorrichtung (1) nach Anspruch 9, **dadurch gekennzeichnet, dass**
die durch den Vortriebskörper (24) und/oder die durch das Sicherungselement (34) gebildete Kavität auf der zum Adapter (44) ausgerichteten Seite durch ein Verschlusselement (28, 38), insbesondere einen Ring (28, 38), verschlossen ist.

11. Austragsvorrichtung (1) nach einem der vorangehenden Ansprüche, **dadurch gekennzeichnet, dass**
die Austragsvorrichtung (1) ein Gehäuse (6) mit einem Handgriff (22) aufweist, wobei vorzugsweise das Sicherungselement (34) fest mit dem Gehäuse (6) verbunden ist und der zumindest eine Vortriebskörper (24) linear beweglich gegen das Gehäuse (6) gelagert ist.

12. Austragsvorrichtung (1) nach einem der vorangehenden Ansprüche, **dadurch gekennzeichnet, dass**
der zumindest eine Vortriebskörper (24) die Klemmstange (2) zumindest bereichsweise umschließt, bevorzugt zu mindestens 75% umschließt, besonders bevorzugt vollständig umschließt.

13. Austragsvorrichtung (1) nach Anspruch 12, **dadurch gekennzeichnet, dass**
der zumindest eine Vortriebskörper (24) eine Durchführung aufweist, die bevorzugt als Röhre ausgebildet ist.

14. Austragsvorrichtung (1) nach einem der Ansprüche 1 bis 11, **dadurch gekennzeichnet, dass**
mehrere axial verschiebbare Vortriebskörper (24) aus unterschiedlichen Richtungen an der Klemmstange (2) anliegen.

15. Austragsvorrichtung (1) nach einem der vorangehenden Ansprüche, **dadurch gekennzeichnet, dass**
die Schneidkanten (54, 56), insbesondere die Federzungen (50, 52) oder das zumindest eine Federplättchen (26, 36), eine Härte von mindestens 45 HRC haben, vorzugsweise von mindestens 50 HRC haben.

16. Austragsvorrichtung (1) nach einem der vorangehenden Ansprüche, **dadurch gekennzeichnet, dass**
die Klemmstange (2) aus einem Kunststoff besteht, bevorzugt aus einem Duroplast.

17. Austragsvorrichtung (1) nach einem der vorangehenden Ansprüche, **dadurch gekennzeichnet, dass**
das zumindest eine Sicherungselement (34) auf der Klemmstange (2) vor oder auch hinter dem zumindest einen Vortriebskörper (24) in Bezug zur Vortriebsrichtung der Klemmstange (2) angeordnet ist.

18. Austragsvorrichtung (1) nach einem der vorangehenden Ansprüche, **dadurch gekennzeichnet, dass**
die Federzungen (50, 52), insbesondere die Federplättchen (26, 36), aus Federstahl bestehen.

19. Verfahren zum Vortreiben einer Klemmstange (2) mit einem manuell zu bedienenden Bedienelement (4) bei dem durch manuelle Krafteinwirkung auf das Bedienelement (4) zumindest ein an der Klemmstange (2) anliegender Vortriebskörper (24) in einer ersten axialen Richtung der Klemmstange (2) bewegt wird,
wobei Schneidkanten (54) mehrerer Federzungen (50) des zumindest einen Vortriebskörpers (24) in die Klemmstange (2) eingreifen und dadurch gegen die Klemmstange (2) klemmen, so dass die Klemmstange (2) mit dem Vortriebskörper (24) in die erste axialen Richtung bewegt wird,
wobei, sobald die manuelle Krafteinwirkung auf das Bedienelement (4) reduziert wird oder beendet wird, der zumindest eine Vortriebskörper (24) durch ein Federelement (40) in eine der ersten axialen Richtung entgegengesetzte zweite axiale Richtung gedrückt wird, wobei das Bedienelement (4) wieder in die Ausgangsstellung überführt wird und wobei sich durch die umgekehrte Krafteinwirkung die Schneidkanten (54) der Federzungen (50) des zumindest einen Vortriebskörpers (24) über die Klemmstange (2) gleiten, und
wobei eine umgekehrte Bewegung der Klemmstange (2) in die zweite axiale Richtung durch zumindest ein an der Klemmstange (2) anliegendes Sicherungselement (34) blockiert wird, wobei hierzu Schneidkanten (56) von Federzungen (52) des Sicherungselements (34) durch die umgekehrte Bewegung der Klemmstange (2) in die Klemmstange (2) eingreifen und dadurch gegen die Klemmstange (2) klemmen, so dass die Klemmstange (2) sich nicht mehr gegen das zumindest eine Sicherungselement (34) bewegt.

20. Verfahren nach Anspruch 19, **dadurch gekennzeichnet, dass** die Oberfläche der Klemmstange (2) durch die Schneidkanten (54, 56) der Federzungen (50, 52) des Vortriebskörpers (24) und des Sicherungselements (34) plastisch verformt wird, wenn die Schneidkanten (54, 56) der Federzungen (50, 52) des Vortriebskörpers (24) und des Sicherungselements (34) in die Klemmstange (2) greifen.

21. Verfahren nach Anspruch 19 oder 20, **dadurch gekennzeichnet, dass** sich bei einer Bewegung der Klemmstange (2) in die erste axiale Richtung die Schneidkanten (56) der Federzungen (52) des Sicherungselements (34) von der Klemmstange (2) aus den durch die Schneidkanten (56) erzeugten Vertiefungen in der Klemmstange (2) lösen und die Klemmstange (2) dadurch gegen das zumindest eine Sicherungselement (34) bewegt wird.

22. Verfahren nach einem der Ansprüche 19 bis 21, **dadurch gekennzeichnet, dass**
beim manuellen Bedienen des Bedienelements (4), insbesondere beim Kippen eines Kipphebels (4) als Bedienelement (4), durch die manuelle Krafteinwirkung das Federelement (40) komprimiert wird.

23. Verfahren nach einem der Ansprüche 19 bis 22, **dadurch gekennzeichnet, dass**
die Bedienung des Bedienelements (4) mehrfach wiederholt wird und dabei die Klemmstange (2) schubweise vorgetrieben wird und dabei schubweise ein Knochenzement aus einer Zementkartusche ausgetrieben wird, die zuvor über einen Adapter (44) mit einer Austragsvorrichtung (1) verbunden wurde, wobei die Austragsvorrichtung (1) den Adapter (44), das Bedienelement (4), die Klemmstange (2), den zumindest einen Vortriebskörper (24) und das Sicherungselement (34) umfasst.

24. Verfahren nach einem der Ansprüche 19 bis 23, **dadurch gekennzeichnet, dass**
das Verfahren unter Verwendung einer Austragsvorrichtung (1) nach einem der Ansprüche 1 bis 17 durchgeführt wird.
